# EUROPEAN PATENT APPLICATION

(11) **EP 1 325 722 A2**
(43) Date of publication of application: **09.07.2003**
(21) Application number: 02027866.9
(22) Date of filing: 12.12.2002
(51) Int. Cl.: A61F 9/013

(54) **Adjustable suction ring**

(30) Priority: 14.12.2001 US 20855
(71) Applicant: Carriazo, Cesar C., Dr., Baranquilla (CO)
(72) Inventor: Carriazo, Cesar C., Dr., Baranquilla (CO)
(74) Representative: Hofstetter, Alfons J., Dr.rer.nat.

(57) **Abstract**

An adjustable suction ring for immobilizing the eyeball for use with any corneal surgical procedure that involves immobilizing the eyeball in relation to surgical tools. The suction ring is adjustable to fit eyeballs of differing diameters. The suction ring consists of an upper portion and a lower portion joined by a threaded connection. By rotating the lower section and backing out the threads, the distance between the upper engaging surface and the lower engaging surface is widened, thereby decreasing the curvature radius of the inferior engaging surface resulting in a proper fit between the suction ring and a small diameter eyeball. By rotating the lower section oppositely, the distance between the upper engaging surface and the lower engaging surface is shortened, thereby effectively increasing the curvature radius of the inferior engaging surface resulting in a proper fit between the adjustable suction ring and a large diameter eyeball.

## Description

This invention relates to surgical apparatus and methods for performing lamellar keratotomies. More particularly, the invention relates to a positioning ring for temporarily immobilizing the eyeball, or ocular globe, such that the globe's cornea protrudes therethrough.

In a normal eye, parallel rays of light entering the eye become focused on the retina to create a sharp visual image. Anomalies in the overall shape of the eye, however, may result in image distortion by causing parallel rays of light entering the eye to become focused at a location other than the retina. Hyperopia, or farsightedness, occurs when the front-to-back measurement of the eyeball is too short, causing parallel rays of light entering the eye to focus behind the retina. In contrast, myopia, or nearsightedness, occurs when the front-to-back measurement of the eyeball is too long, causing parallel rays of light entering the eye to focus in front of the retina. Astigmatism occurs when the parallel rays of light entering the eye do not focus at a single point in the eye, but rather have a variable focus due to an aspherical cornea refracting light in a different meridian at different distances.

Glasses or contact lenses usually correct hyperopia, myopia and astigmatism but surgical corrective methods have become quite popular due to the inconvenience and discomfort of wearing glasses or contact lenses. One of these surgical corrective methods is laser-assisted in-situ keratomileusis (LASIK). During the LASS procedure, a microkeratome is used to perform an incomplete lamellar keratotomy, which leaves a peripheral residue of corneal tissue uncut to act as a hinge. The hinge permits the corneal disk to be lifted for exposing and resecting the stromal layer with a laser. The tissue removed by the laser reshapes the stromal layer so that the stromal layer will sufficiently refract the light rays entering the eye to cause them to focus on the retina, producing a sharp visual image without the aid of glasses or contact lenses. After the laser completes the reshaping of the stromal layer, the corneal disk is folded back into its original position, using the hinge as a guide. Within minutes, the corneal disk adheres itself to the rest of the cornea and the LASIK procedure is complete.

There are many different designs for microkeratomes but certain aspects of their operation are similar. A suction ring is first affixed to the sclera and centered about the cornea so that the cornea extends through an aperture and above the suction ring. The diameter of the aperture in the suction ring through which the cornea extends is selected on the basis of the size of the cornea and the diameter of the corneal disk to be cut. The suction ring is held to the sclera by a vacuum induced in the area between the cylindrical ring and the eyeball. A float head having a flat, arcuate or oblique surface is then moved over the guide ring so as to compress the cornea into a shape that complements the surface. A cutting head carrying a blade is then moved across the suction ring so as to resect a corneal disk. The float head may be held stationary once it has compressed the cornea prior to the movement of the blade, or the float head may be moved with the blade head while maintaining contact with the cornea. The cutting blade is moved a predetermined distance substantially, but not completely, across the portion of the cutting plane that intersects the cornea. The movement of the cutting blade is restricted by an adjustable stop means, whereby the extent of hinge width formed on the corneal disk is adjustable. The cutting blade is then moved back across the suction ring to its original position, so that the suction ring can be removed and the resulting corneal disk can be folded back and secured over its hinge, exposing the corneal stroma to be reshaped by the laser.

The suction ring immobilizes the eye in relation to the microkeratome and further provides a fixed support along which the blade head slides to resect the corneal disc with the blade. Both of these functions are critical and failure of either may result in severe eye damage if the eye is not held steady during the cutting procedure. For example, if the suction ring is not firmly attached to the eyeball, an irregular cut may result causing irregular astigmatism in the patient's eye. Additionally, the disk may be cut off entirely, instead of being fixed by the hinge, or a buttonhole cut may result. In a worse case scenario, the eyeball itself may be punctured resulting in major complications to the health of the eye.

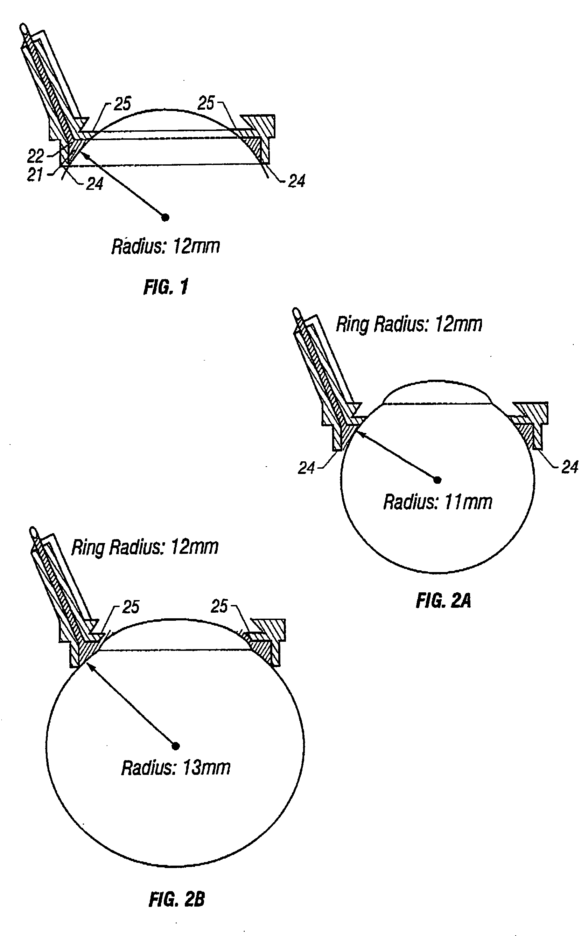

The suction ring grips the eyeball through suction applied by a small vacuum pump or other vacuum source. The suction ring must be securely affixed to the sclera and the corneal region by the suction induced by the vacuum source to immobilize the eyeball and provide the required support. The suction ring is a cylinder with a circular aperture on the top end through which the cornea is exposed for cutting. The bottom end of the suction ring is also circular and attaches to the sclera. The suction induced by a vacuum is normally pulled through ports around the inside wall of the suction ring. To acquire the necessary suction to hold the suction ring in place on the eye, a seal must be maintained both around the bottom end of the suction ring with the sclera as well as the top aperture of the suction ring with the corneal region. Figure **1** shows a properly fitted suction ring on an eye. A good suction seal is formed between the corneal region and the top aperture **25** and between the sclera and the bottom end of the suction ring **24.** The suction chamber **21** is the volume inside the cylinder formed by the suction ring and sealed by the eye. The vacuum is induced through a port **22** to the vacuum source.

Because of the differing diameters of eyeballs, the suction ring in use today does not always properly grip the eyeball. As shown in FIG. **2A**, a particular suction ring attached to a small eyeball may not form an adequate seal between the bottom end of the suction ring **24** and the sclera. As shown in FIG **2B**, a particular suction ring attached to a large eyeball may not form an adequate seal between the corneal region and the top aperture **25**. Often, the attempt is made to solve these problems by applying higher vacuum and sucking the eyeball into place to form the required seal. However, using the higher vacuum increases the intraocular pressure and places the eye structures, such as the retina, at risk. Alternatively, kits of differing sized rings are available so that a ring having a diameter closer to the diameter of the eyeball undergoing the procedure may be used.

Therefore, there is a need for a suction ring that ensures a good contact leading to a good suction seal both around the top aperture and around the sclera for eyeballs of differing diameters. It would be very advantageous to have a microkeratome that greatly reduced the risk of suction loss between the eyeball and the suction ring while performing an incomplete lamellar keratotomy procedure on eyeballs of differing diameters. It would be very desirable to have a single ring that would achieve an improved fit on eyeballs of various sizes. It would also be desirable if the surgeon could adjust the fit without using additional rings.

The present invention provides an adjustable suction ring that is suitable for use with a microkeratome to secure an ocular globe during an incomplete lamellar keratotomy procedure. Alternatively, the present invention could be used for any procedure that involves immobilizing the eyeball in relation to surgical tools for a surgical procedure on the cornea. The adjustable suction ring comprises an upper section containing an aperture sized to receive and expose a cornea, a lower section extendably coupled to the upper section, and an annular vacuum channel that is connectable to a vacuum source, wherein the annular vacuum channel has an ocular globe-engaging surface comprising an inferior engaging surface and a superior engaging surface. The lower section has outer threads, the upper section has inner threads, and the lower section is connected to the upper section by mating the outer threads with the inner threads. By rotating the lower section, the lower section is extended in relation to the upper section by rotating the lower section in a first direction and is retracted in relation to the upper section by rotating the lower section in a second direction. Alternatively, the lower section may have inner threads and the upper section outer threads.

Extending and retracting the lower section in relation to the upper section generates a higher and lower curvature radius for the inferior engaging surface, thereby providing a suitable suction seal for ocular globes of varying diameters.

The aperture is a shape selected from the group consisting of circular, elliptical, oval, and ovoid. The inferior and superior engaging surfaces may be defined by a plurality of meridians having different radii or alternatively, by a plurality of meridians having equal radii. The inferior and superior engaging surfaces each has a shape selected from the group consisting of circular, elliptical, oval, ovoid, and combinations thereof.

The present invention also provides a microkeratome having an adjustable suction ring.

The foregoing and other objects, features and advantages of the invention will be apparent from the following more particular description of a preferred embodiment of the invention, as illustrated in the accompanying drawing wherein like reference numbers represent like parts of the invention.

FIG. **1** is a cross-sectional view of a suction ring properly fitted to an eyeball.

FIGs. **2 A-B** are cross-sectional views of poorly fitted suction rings on eyeballs of differing diameters.

FIG. **3** is a perspective view of an adjustable suction ring.

FIGs. **4 A-B** are cross-sectional views of the adjustable suction ring fitted to eyeballs of differing diameters.

The present invention provides an adjustable suction ring that is suitable for use with a microkeratome during an incomplete lamellar keratotomy procedure. Alternatively, the present invention could be used for any procedure that involves immobilizing the eyeball in relation to surgical tools for a surgical procedure on the cornea.

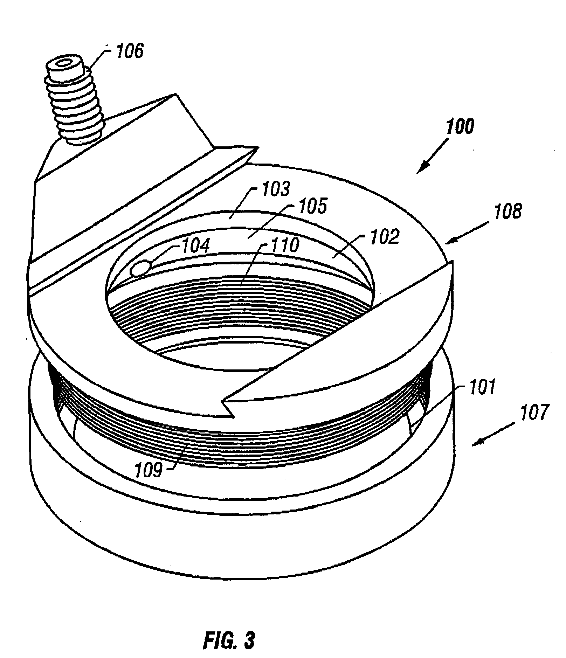

FIG. **3** presents a perspective view of an adjustable suction ring of the present invention. The suction ring **100** presents an aperture **105** into which the cornea is received. A tube connection **106** is used to connect the suction ring through a tube to a vacuum source, such as a vacuum pump not shown, to provide suction through a suction port **104** in the annular vacuum channel **102**. The inferior engaging surface **101**, which is the inferior inner wall of the lower section **107** of the suction ring, is placed on the sclera and the superior engaging surface **103**, which is the superior inner wall of the upper section **108** of the suction ring, engages the corneal region. The engaged corneal region is the cornea and a portion of the limbo, the zone that joins the cornea and the conjunctiva. The upper threads **109** on the upper section of the suction ring mate with the lower threads **110** on the lower section of the suction ring. By rotating the lower section **107**, the lower section can be raised and lowered in relation to the upper section 108. When suction is applied to the annular vacuum channel **102**, the ocular globe is slightly drawn into the annular vacuum channel, or "pinched", creating a seal and firmly gripping and immobilizing the eye in relation to the microkeratome.

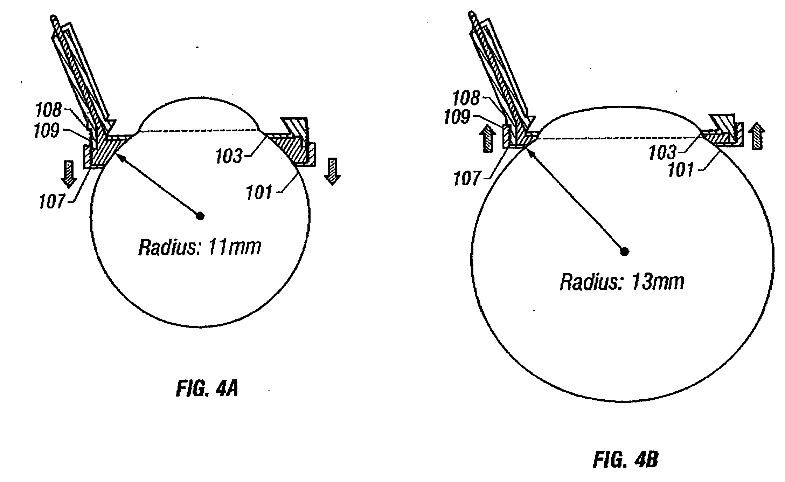

FIGs. **4 A-B** show cross-sectional side views of the adjustable suction ring engaging eyeballs of differing diameters. In FIG. **4 A,** the lower section **107** has been rotated to back out the threads **109**, thereby extending the lower section **107** downward in relation to the upper section **108**. Since the distance between the inferior engaging surface **101** and the superior engaging surface **103** is relatively far apart, the effective lower curvature radius of the inferior engaging surface is lower, resulting in a good seal between the engaging surfaces **101, 103** and the small diameter eyeball. Alternatively, in FIG. **4 B,** the lower section **107** has been rotated to take in the threads **109,** thereby moving the lower section **107** upward in relation to the upper section **108**. Since the distance between the inferior engaging surface **101** and the superior engaging surface **103** is relatively close together, the effective lower curvature radius of the inferior engaging surface is higher, resulting in a good seal between the engaging surfaces **101, 103** and the large diameter eyeball.

There are currently many different suction rings, or guide rings as they are also called, in use today differing mainly in the means for securing the microkeratome to the suction or guide ring. The adjustability of the present invention may be applied equally successfully to all these types of rings and any rings that may be developed in the future. The benefit of the suction ring of the present invention is that one ring may be used to properly fit eyeballs of widely differing diameters, without risking injury to the structures of the eye by increasing the suction pressure required to secure the suction ring to the eyeballs.

While the present invention provides an upper section having outer threads and a lower section having inner threads used to couple the two sections, alternatively the upper section could have inner threads and the lower section outer threads without changing the scope of the invention. Furthermore, any means for joining the upper and lower section would be adequate as long as the means allowed for adjustment of the distance between the superior engaging surface and the inferior engaging surface to account for the differing eyeball diameters used by the suction ring. For example, optionally a bellows arrangement could be used, having adjustable supports that would allow the lower section to be moved relative to the upper section with the bellows providing the airtight seal required by the suction chamber to securely attach the suction ring to eyeballs of differing diameters.

Furthermore, the present invention is not limited only to cylinders with circular apertures for the corneal region and circular inferior engaging surfaces for gripping the sclera. Optionally, the aperture of the suction ring may be circular, elliptical, oval or ovoid. Additionally, the inferior and superior engaging surfaces may be circular, elliptical, oval, ovoid or combinations thereof. If the lower and upper sections are joined with threads, then the threaded area must be cylindrical. However, the engaging areas may be machined or formed in a different shape. Alternatively, if a different coupling means is used, for example, use of bellows and clamps, the coupling area would not be required to be cylindrical.

The present invention also includes a microkeratome using the adjustable suction or guide ring. A microkeratome is a surgical instrument used for surgical procedures such as, for example, lamellar keratotomies. Microkeratomes are well known by those having ordinary skill in the art and are fully described by Carriazo in U.S. Patent No. 6,296,650 and by Carriazo, et al. in U.S. Pat. No. 5,980,543, both patents hereby being fully incorporated herein by reference.

## Claims

1. A suction ring for securing an ocular globe, comprising:
an upper ring section containing an aperture sized to receive and expose a cornea;
a lower ring section extendably coupled to the upper ring section;
an annular vacuum channel that is connectable to a vacuum source, wherein the annular vacuum channel has an ocular globe-engaging surface comprising an inferior engaging surface and a superior engaging surface.

2. The apparatus of claim 1, wherein the lower ring section has outer threads, wherein the upper ring section has inner threads, and wherein the lower ring section is connected to the upper ring section by mating the outer threads with the inner threads.

3. The apparatus of claim 2, wherein the lower ring section is extended in relation to the upper ring section by rotating the lower ring section in a first direction, wherein rotating the lower ring section in the first direction increases the number of mated outer and inner threads.

4. The apparatus of claim 3, wherein the lower ring section may be retracted in relation to the upper ring section by rotating the lower ring section in a second direction, wherein rotating the lower ring section in the second direction decreases the number of mated outer and inner threads.

5. The apparatus of claim 1, wherein the lower ring section has inner threads, wherein the upper ring section has outer threads, and wherein the lower ring section is connected to the upper ring section by mating the outer threads with the inner threads

6. The apparatus of claim 1, wherein the lower ring section may be extended and retracted in relation to the upper ring section, wherein retracting the lower ring section generates a lower curvature radius for the inferior engaging surface and wherein extending the lower ring section generates a higher curvature radius for the inferior engaging surface.

7. The apparatus of claim 1, wherein the aperture is a shape selected from the group consisting of circular, elliptical, oval, and ovoid.

8. The apparatus of claim 1, wherein the inferior engaging surface and the superior engaging surface are defined by a plurality of meridians having different radii.

9. The apparatus of claim 1, wherein the inferior engaging surface and the superior engaging surface are defined by a plurality of meridians having equal radii.

10. The apparatus of claim 1, wherein the inferior and superior engaging surfaces each has a shape selected from the group consisting of circular, elliptical, oval, ovoid and combinations thereof.

11. The apparatus of claim 1, wherein the suction ring is made from a material selected from the group consisting of stainless steel, titanium, synthetic plastic, rubber and combinations thereof.

12. A microkeratome for performing a lamellar keratotomy of an aspherical ocular globe, comprising:
a suction ring comprising an upper ring section containing an aperture sized to receive and expose a cornea, a lower ring section extendably coupled to the upper ring section, an annular vacuum channel that is connectable to a vacuum source, wherein the annular vacuum channel has an ocular globe-engaging surface comprising an inferior engaging surface and a superior engaging surface;
a blade suitable for corneal resections;
a cutting head for carrying the blade over the suction ring through a cutting path defined by the suction ring;
an adjustable cornea compression device connected to the cutting head for at least partially compressing the cornea ahead of the blade so as to set the corneal resection to a desired shape and thickness;
means for driving the cutting head and the cornea compression device across the suction ring.

13. The microkeratome of claim 12, wherein the lower ring section has outer threads, wherein the upper ring section has inner threads, and wherein the lower ring section is connected to the upper ring section by mating the outer threads with the inner threads.

14. The microkeratome of claim 13, wherein the lower ring section is extended in relation to the upper ring section by rotating the lower ring section in a first direction, wherein rotating the lower ring section in the first direction increases the number of mated outer and inner threads.

15. The microkeratome of claim 14, wherein the lower ring section may be retracted in relation to the upper ring section by rotating the lower ring section in a second direction, wherein rotating the lower ring section in the second direction decreases the number of mated outer and inner threads.

16. The microkeratome of claim 12, wherein the lower ring section has inner threads, wherein the upper ring section has outer threads, and wherein the lower ring section is connected to the upper ring section by mating the outer threads with the inner threads.

17. The microkeratome of claim 12, wherein the lower ring section may be extended and retracted in relation to the upper ring section, wherein retracting the lower ring section generates a lower curvature radius for the inferior engaging surface and wherein extending the lower ring section generates a higher curvature radius for the inferior engaging surface.

18. The microkeratome of claim 12, wherein the aperture is a shape selected from the group consisting of circular, elliptical, oval, and ovoid.

19. The microkeratome of claim 12, wherein the inferior engaging surface and the superior engaging surface are defined by a plurality of meridians having different radii.

20. The microkeratome of claim 12, wherein the inferior engaging surface and the superior engaging surface are defined by a plurality of meridians having equal radii.

21. The microkeratome of claim 12, wherein the inferior and superior engaging surfaces each has a shape selected from the group consisting of circular, elliptical, oval, ovoid and combinations thereof.

22. The microkeratome of claim 12, wherein the suction ring is made from a material selected from the group consisting of stainless steel, titanium, synthetic plastic, rubber and combinations thereof.

23. The microkeratome of claim 12, wherein the cutting path is horizontal.

24. The microkeratome of claim 12, wherein the cutting path is pendular.
